(19) Europäisches Patentamt
European Patent Office
Office européen des brevets

(11) Publication number: **0 011 707**
**B1**

(12) # EUROPEAN PATENT SPECIFICATION

(45) Date of publication of patent specification: **15.09.82**

(21) Application number: **79104073.6**

(22) Date of filing: **22.10.79**

(51) Int. Cl.³: **C 07 F 17/00**, C 07 F 7/28, C 08 F 138/02, C 07 C 2/34, C 07 C 11/08

(54) **Lithium dicyclopentadienyl titanium hydride compositions, a process for producing them and their use as catalysts in the production of 1-butene and in polymerizing acetylene.**

(30) Priority: **03.11.78 US 957392**
**03.11.78 US 957592**

(43) Date of publication of application:
**11.06.80 Bulletin 80/12**

(45) Publication of the grant of the patent:
**15.09.82 Bulletin 82/37**

(84) Designated Contracting States:
**BE DE FR GB IT NL**

(56) References cited:
**US - E - 3 776 932**

**ANGEWANDTE CHEMIE, vol. 80, no. 10 page 398, 1968**
**JOURNAL OF THE AMERICAN CHEMICAL SOCIETY, vol. 89, 1967, page 687, H. H. BRINTZINGER: "Hydride, Alkyl, and Allyl Complexes of Bis (n-cyclopentadienyl) titanium (III)"**

(73) Proprietor: **ALLIED CORPORATION**
**Columbia Road and Park Avenue P.O. Box 2245R (Law Dept.)**
**Morristown New Jersey 07960 (US)**

(72) Inventor: **Pez, Guido P.**
**7 Lakeview Avenue**
**Boonton New Jersey 07005 (US)**

(74) Representative: **Collier, Jeremy Austin Grey et al,**
**J.A.Kemp & Co. 14, South Square Gray's Inn**
**London WC1R 5EU (GB)**

**EP 0 011 707 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Lithium dicyclopentadienyl titanium hydride compositions, a process for producing them and their use as catalysts in the production of 1-butene and in polymerizing acetylene

## Background of the invention

### 1. Field of the invention

This invention relates to novel lithium dicyclopentadienyl titanium hydride compositions, useful as catalysts especially in the dimerization of ethylene to 1-butene and in the polymerization of acetylene; and to processes for their synthesis.

### 2. Brief description of the background of the invention including prior art

Cyclopentadienyl titanium complexes and nitrogen adduct complexes thereof are described in the following references: USP 3,776,932 (1973), RE 29,368 (1977), USP 4,024,169 (1977), and *J. Amer. Chem. Soc.*, *98*, 8072—8078 (1976), by Guido Pez (said patents being assigned to Allied Chemical Corp.), hereby incorporated by reference. Per the references, these complexes are capable of converting ethylene to mixtures of ethane, butane, 1-butene and 1,3-butadiene. In a literature article of S. L. Hsu et al. (J. Chem. Phys. *69* 106—111 of July, 1978) use is described of such titanium complexes, in hexane solution, for polymerization of acetylene by contacting acetylene gas with this solution.

G. Henrici et al., *Angewandte Chemie,* vol. 80, pp. 398—99 (1968) discloses compositions with lithium or sodium cations associated with dicyclopentadienyl titanium dihydride anions. An alternate formula shows lithium cations associated with dititanium tetracyclopentadienyl dihydride anions. H. H. Brintzinger, *J. Amer. Chem. Soc.*, vol 89, pp. 6871—76 (1967) also discloses dicyclopentadienyl titanium dihydride amions with which lithium countinuous may be associated. A monohydride complex is also proposed in this reference, but as a neutral species without an associated lithium cation.

## Summary of the invention

We have found a new class of organotitanium compositions, namely, lithium dicyclopentadienyl titanium hydrides, which can be conveniently prepared, and are effective in particular as catalysts of dimerization of ethylene to 1-butene, and of polymerization of acetylene.

In accordance with this invention, there is provided an organotitanium composition characterized by the formula: LiRR'TiH, wherein R and R' are cyclopentadienyl rings of the empirical formula, $C_5H_5$, or are such rings wherein one or more of the ring hydrogens is substituted with a substituent inert toward lithium metal, said rings being bonded to titanium by sigma-bonds, pi-bonds and/or mixtures thereof, and said composition exhibiting a $^{13}C$ nuclear magnetic resonance spectrum in deuterated benzene, in which observed values for the chemical shifts of the ring carbons in R are different from the observed values for ring carbons in R', and said composition exhibiting an infrared spectrum in deuterated n-hexadecane in which a titanium metalhydride absorption band is observed in the region of about 1250—1450 $cm^{-1}$.

Also provided is a process for producing the subject compositions characterized by contacting metallic lithium with a dititanium complex of the formula:

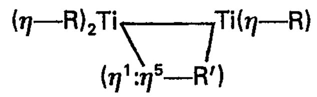

wherein the ($\eta$—R) radicals are cyclopentadienyl rings, bonded to titanium through five carbon atoms each having the empirical formula, $C_5H_5$, or are such rings wherein one or more of the ring hydrogens is substituted with a substituent inert toward lithium metal, and wherein the ($\eta^1$:$\eta^5$—R') radical is a cyclopentadienyl ring, bonded to titanium by a mixture of $\eta^1$ and $\eta^5$ bonds, and having the empirical formula, $C_5H_4$, or is such ring wherein one or more of the ring hydrogens is substituted with a substituent inert toward lithium metal; in a inert solvent therefor and in the presence of a polynuclear aromatic hydrocarbon solubilizing agent for lithium, soluble in said solvent; and at a temperature of about −10° to +60°C, in the absence of elemental oxygen and water; and with initial molar ratio of metallic lithium to said dititanium complex of 10:1 to 1:1.

Further provided is a second process for producing the subject compositions characterized by contacting metallic lithium with a titanium compound of the formula: $R_2TiX_a$, or dimers thereof, wherein the radicals R are cyclopentadienyl rings, each having the empirical formula, $C_5H_5$, or are such rings wherein one or more of the ring hydrogens is independently substituted with a substituent inert toward lithium metal; X being halogen, and the subscript *a* being one or two; in diethyl ether solvent and in the presence of a polynuclear aromatic hydrocarbon solubilizing agent for lithium, soluble in diethyl ether; and at a temperature of −80° to +80°C, in the absence of elemental oxygen and water; and with initial molar ratio of metallic lithium to said titanium compound of 10:1 to 3:1.

Also provided is a process for producing 1-butene comprising contacting ethylene gas with a solution of dimetrization catalyst, in an inert solvent therefor, under pressure of 1 to 100 atmospheres, at a temperature 0° to 100°C; characterized by providing the subject organotitanium composition of formula LiRR'TiH

described hereinabove as said catalyst; and also a process for polymerizing acetylene, comprising contacting acetylene gas under essentially anhydrous, oxygen-free conditions, with a solution consisting essentially of an organic solvent and a dissolved organotitanium catalyst composition at a temperature in the range between minus 20°C and plus 200°C, characterized by providing the subject organotitanium composition of formula LiRR'TiH described hereinabove as said catalyst.

Brief description of the drawings

Figure 1 is a $^{13}C$ nuclear magnetic resonance spectrum taken at 20 MHz, of

$$Li(\eta{-}C_5H_5)(C_5H_5)Ti{-}H$$

in deuterated benzene containing tetramethylethylenediamine. Chemical shifts are in ppm versus tetramethylsilane (calculated via $C_6D_6$).

Figure 2 is an infrared spectrum of crystalline

$$Li(\eta{-}C_5H_5)(C_5H_5)Ti{-}H \cdot (CH_3OCH_2CH_3OCH_3)$$

adduct in a mull of deuterated n-hexadecane between two KBr disks. (The peaks marked with an asterisk are due to the mulling agent).

Description of the invention and preferred embodiments

The novel lithium titanium hydride compositions of this invention are soluble in conventional organic solvents which are commonly used in catalytic reactions and processes. The structure of the subject compositions in terms of bonding characteristics and spatial geometry is not yet accurately known since a sufficiently well-ordered single crystal is not yet available. However, by analogy to the structure of various "titanocene" type compounds, described in *J. Amer. Chem. Soc., 98*, 8072—8078 (1976), and from known bonding characteristics of other titanium complexes it can be hypothesized that the bonding of the two cyclopentadienyl rings to the titanium metal involves sigma bonds, pi bonds and/or mixtures thereof. By the term "pi-bond", designated in this application as $\eta^5$ using IUPAC nomenclature (International Union of Pure and Applied Chemistry), is meant a covalent bond formed between a titanium atom and the cyclopentadienyl ring by electrons, denoted by the respective titanium and five carbon atoms, moving in orbitals extending above and below the plane of the cyclopentadienyl ring. By the term "sigma-bond" designated as $\eta^1$ using IUPAC nomenclature, is meant a covalent bond formed between a titanium atom and one carbon atom of a cyclopentadienyl ring by electrons, donated by the respective atoms, and directed along the line joining the centers of the two atoms. The term "mixtures" thereof, as used herein, includes hybrid bonding between a titanium atom and 2, 3 or 4 carbon atoms of a cyclopentadienyl ring which is termed $\eta^2$, $\eta^3$, $\eta^4$ bonding, respectively, by accepted IUPAC nomenclature. The term "mixture thereof" also encompasses bonding which is distinctly a mixture of sigma and pi bonding and designated in this application as $\eta^1{:}\eta^5$ bonding. Where the number of carbon atoms of the dienyl ring attached to the metal atom is obvious from the stated formula, no superscript is used with the nomenclature; thus, for example,

$$Li(\eta^5{-}C_5H_5)(C_5H_5)TiH$$

and

$$Li(\eta{-}C_5H_5)(C_5H_5)TiH$$

are equivalent. The symbol "$\mu$" designates (IUPAC) a bridging structure, here a cyclopentadienyl ring bridging two titanium atoms.

Certain physical properties of the subject compositions, as determined spectroscopically, clearly indicate that the two cyclopentadienyl rings are not equivalent and that they are differently bonded to titanium. A bonding difference is evidenced by the proton decoupled $^{13}C$ nuclear magnetic resonance (NMR) spectrum of the unsubstituted compound,

$$Li(\eta{-}C_5H_5)(C_5H_5)Ti{-}H,$$

in deuterated benzene, containing tetramethylethylenediamine to aid in solubilizing the compound, as illustrated in the lower curve of Fig. 1, wherein the observed values for the chemical shifts of the five ring carbons of one cyclopentadienyl ring, designated as ($\eta{-}C_5H_5$), are all equivalent and occur in one combined absorption peak at 95.21 ppm downfield vs. tetramethylsilane standard, TMS, taken as 0 ppm. The observed values for the chemical shifts of the ring carbons in the other cyclopentadienyl ring, designated as ($C_5H_5$), occur at five non-equivalent positions ranging from 100.93 to 111.36 ppm relative to TMS standard. The upper curve of Fig. 1 illustrates the off-resonance protoncoupled, $^{13}C$ NMR spectrum which shows that there is one hydrogen atom per each carbon atom in each of the cyclopentadienyl rings ($C_5H_5$ and $\eta{-}C_5H_5$). This technique for determining the structural and bonding equivalency and nonequivalency of carbon atoms in organic molecules is well known and accepted in the art.

By the term "substituted with a substituent inert toward lithium metal" as used herein and applied to the cyclopentadienyl rings, is meant that each of the five ring hydrogens of each cyclopentadienyl ring may independently be substituted by a substituent which is inert toward lithium metal, under the conditions of solution in an inert organic solvent at room

temperature. This requirement of inertness toward reaction with lithium metal stems from the fact that the subject compositions are generally prepared from the precursor cyclopentadienyl titanium compounds, described in the above references by Guido Pez, by reaction with lithium metal. Thus, the term does not embrace substituent groups which are replaceable by reaction with lithium, such as the halogens or groups containing active hydrogen, such as hydroxy. Representative examples of suitable substituents include $C_1$—$C_4$ linear or branched alkyl, $C_1$—$C_4$ linear or branched alkoxy, and phenyl. Where the cyclopentadienyl rings are substituted, preferred substituents are the above-described $C_1$—$C_4$ alkyl, and particularly methyl. Where the cyclopentadienyl ring is substituted, it is preferably monosubstituted.

The hydride character of the subject composition resides in the titanium metal-hydride bond, i.e. Ti—H. By the term "hydride", as used herein, is meant that the hydrogen attached to the titanium metal atom in the subject composition can support a negative charge. Evidence for the metal-hydride bond in the subject compositions is provided by the fact that they exhibit an infrared spectrum, in a mull of deuterated n-hexadecane, between two potassium bromide discs, which contains an absorption band at about 1250 to 1450 $cm^{-1}$, which is generally broad and intense. The exact position of the band within this range is largely dependent upon the nature of the solvent used to solvate the lithium cation in the resulting complex. Thus, for crude

$$Li(\eta—C_5H_5)(C_5H_5)Ti—H,$$

obtained from the first described process herein using diethyl ether as solvent, the Ti—H absorption band occurs at 1390 $cm^{-1}$; whereas in a crystalline form of the compound prepared in 1,2-dimethoxyethane as solvent, the Ti—H absorption band occurs at 1315 $cm^{-1}$, as illustrated in Fig. 2. Substituents on the cyclopentadienyl rings, as defined herein, will not significantly change the region of absorption of the metal-hydride band outside of this stated region.

Further evidence for the presence of the metalhydride bond in the subject compositions is furnished by reaction of the subject compositions with methyl iodide wherein it is found that approximately one mole of methane is liberated by reaction of at least one mole of methyl iodide with one mole of subject composition. This technique is well known and accepted in the art as evidence of the presence of a hydride moiety.

Since the subject compositions contain a lithium cation, the compositions are readily soluble in solvents which act as chelating agents for the lithium cation by virtue of the fact that the solvents contain atoms having unshared non-bonding electrons, such as oxygen and nitrogen atoms. Representative examples include diethyl ether, 1,2-dimethoxyethane (DME), 1,2-diethoxyethane, p-dioxane, tetrahydrofuran, crown ethers such as 15-crown-5 (1,4,7,10,13-pentaoxycyclopentadecane), 18-crown-6 (1,4,7,10,13,16-hexaoxycyclooctadecane), dibenzo and dicyclohexyl derivatives thereof, diamines such as N,N,N',N'-tetramethylethylenediamine, and cryptates, being bicyclic bridgehead diamines, having oxymethylene bridges, and the like. It is to be understood that adducts formed between the subject compositions and organic compounds, processing the ability to chelate lithium, are encompassed as compositions within the scope of this invention. Adducts formed from other chelating agents, not specifically described herein, will also be equivalent and obvious to one skilled in the art.

Other physical properties of the compositions in addition to the observed $C^{13}$ nuclear magnetic resonance behavior and infrared spectrum include a reddish brown color of the solid compositions and a proton nuclear magnetic resonance spectrum which shows a metalhydride resonance at −4.64 ppm, upfield from tetramethylmethylsilane.

Representative examples of the compositions of the invention include

$$Li(CH_3—C_5H_4)(\eta—C_5H_5)Ti—H,$$
$$Li(C_5H_5)(\eta—CH_3—C_5H_4)Ti—H,$$
$$Li(\eta—CH_3—C_5H_4)(\eta—CH_3—C_5H_4)Ti—H,$$

and

$$Li([\eta—C_5(CH_5)][C_5(CH_5)_5]Ti—H.$$

Preferred composition is

$$Li(\eta—C_5H_5)(C_5H_5)Ti—H,$$

useful as a catalyst in converting ethylene to 1-butene and in polymerization acetylene.

Also a subject of this invention is a process for preparing the subject compositions in which lithium metal is contacted in a solution in inert solvent in the presence of a polynuclear aromatic hydrocarbon solubilizing agent for lithium, at a temperature of about −10° to +60°C in the absence of elemental oxygen and water with dititanium complex, in initial molar ratio of metallic lithium to dititanium complex of about 10:1 to 1:1, wherein. the dititanium complex has the formula:

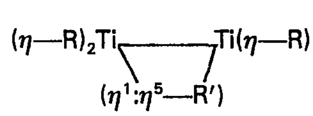

The (η—R) radicals in the above formula are cyclopentadienyl rings, bonded to Ti through five carbon atoms, having the empirical formula, $C_5H_5$, or such rings wherein one or more of the cyclopentadienyl ring hydrogen atoms is substituted with a substituent inert toward lithium metal, said substituents being identical in

nature to those described hereinabove for the subject compositions. The ($\eta^1$:$\eta^5$—R') radical is a cyclopentadienyl ring, bonded to titanium by a mixture of $\eta^5$ and $\eta^1$ bonds and having the empirical formula, $C_5H_4$, wherein one or more of the cyclopentadienyl ring hydrogens may be substituted with a substituent inert toward lithium metal as described hereinabove.

An exact description of the nature of the bonding in these precursor cyclopentadienyl dititanium compounds is known in the art and described in *J. Amer. Chem. Soc., 98,* 8072—8078 (1976), hereby incorporated by reference. Representative examples of cyclopentadienyl dititanium compounds useful in the process are the parent compound, $\mu$ - ($\eta^1$:$\eta^5$ - cyclopentadienyl) - tris($\eta$ - cyclopentadienyl)dititanium (Ti—Ti), and substituted derivatives thereof, wherein the derivatives are formed by substituting the ring hydrogens on the respective cyclopentadienyl rings with said substituents as described hereinabove, for the subject compositions. Preferred compound in the process is the above-described ring-unsubstituted parent compound.

The polynuclear aromatic hydrocarbon solubilizing agent for lithium, soluble in said solvent, is essential for success of the reaction and includes naphthalene, anthracene, biphenyl and the like. A preferred agent is naphthalene. It is believed that the solubilizing agent reacts with lithium in the process to form a lithium cation aromatic anion complex, a "metal arene", which thus aids the efficiency of the reaction process. The amount of solubilizing agent used is generally about 1 to 10 parts by weight per part of lithium metal and preferably about 1 to 2 parts by weight of lithium metal used.

The molar ratio of metallic lithium to said dititanium complex used is generally about 10:1 to 1:1 and preferably about 7:1.

The process is generally conducted in the temperature range of about −10 to +60°C and preferably at about 20—25°C.

Solvents useful in the process include those which have good solvating ability for the titanium compounds and include $C_2$—$C_6$ saturated aliphatic monoethers, $C_2$—$C_6$ linear saturated aliphatic diethers, $C_4$—$C_6$ cyclic saturated aliphatic monoethers, and $C_4$—$C_6$ cyclic saturated aliphatic diethers. Representative examples include diethyl ether, tetrahydrofuran, dioxane, 1,2-dimethoxyethane and the like. Preferred solvent is diether ether.

The amount of solvent used is generally about 100 to 500 parts by weight per part of titanium compound, although not limited thereto.

The process must be conducted in the absence of elemental oxygen and water to minimize decomposition of the desired product and degradation or hydrolysis of the starting dititanium complex.

Various forms of apparatus may be used for the reaction and one which the inventor found useful was a hollow evacuable H-shaped glass apparatus having elongated vertical cylindrical sides connected by a horizontal tube of the same internal diameter as the vertical sides. An internal glass filter, positioned in the horizontal tube was used to filter the reaction product. Each bottom portion of the vertical sides of the H-tube, in an upright position, can serve as a reaction vessel in which the contents can be conveniently heated or cooled or stirred by the introduction of a magnetic stirring bar therein. The H-shaped apparatus can be used in a conventional dry box, which can be evacuated, or filled with argon, such that the entire reaction can be conducted under conditions in the absence of elemental oxygen and water.

Generally, dry lithium powder, a dititanium complex and inert solvent, such as diethyl ether, and a polynuclear aromatic hydrocarbon such as naphthalene are placed into one of the vertical sides of the H-shaped apparatus, under a dry inert atmosphere, and the mixture is stirred overnight at room temperature. The resulting mixture is then filtered by appropriately tipping the H-shaped apparatus such that the contents of the vertical tube are constrained to pass downward through the horizontal tube containing the glass filter frit. The remaining solid is repeatedly extracted with additional solvent.

The solvent extracts and filtrate are combined and the solvent removed, in vacuo, resulting in a product composition of a purity in the range of about 60 to 80%. Purification can be accomplished in the apparatus by recrystallization from a suitable solvent such as diethyl ether or 1,2-dimethoxyethane.

Yields of the lithium titanium hydride composition are in the range of about 25% of theory based on starting dititanium complex.

In an alternative process of this invention for synthesizing the lithium titanium hydride compositions of this invention, metallic lithium is contacted with a titanium compound of the formula: $R_2TiX_a$, or dimers thereof, wherein the radicals R are cyclopentadienyl rings, each having the empirical formula, $C_5H_5$, or are such rings wherein one or more of the ring hydrogens is substituted with a substituent inert toward lithium metal, said groups being defined and described hereinabove; X being halogen, such as fluorine, chlorine, bromine or iodine, and *a* being one or two. The process is conducted in a solvent, specifically diethyl ether, for the titanium compound and in the presence of a polynuclear aromatic hydrocarbon solubilizing agent for lithium, said agent also being soluble in the diethyl ether solvent; and in the absence of elemental oxygen and water, at a temperature of about −80°C to +80°C, and with an initial molar ratio of metallic lithium to said titanium compound of about 10:1 to 3:1.

The titanium compounds useful in this alternative process include dicyclopentadienyl titanium monochloride and dichloride, dicyclopentadienyl titanium monobromide and

dibromide, di(3-methylcyclopentadienyl) titanium monochloride and dichloride, and the like. Preferred starting titanium compounds are the dichloride and dibromide of dicyclopentadienyl titanium.

Diethyl ether was the only solvent studied found to be effective in carrying out the invention process. Tetrahydrofuran and 1,2-dimethoxyethane were found to be inoperative. The amount of diethyl ether solvent used is generally about 100 to 500 parts by weight par part of said dititanium complex, although not limited thereto.

A polynuclear aromatic hydrocarbon solubilizing agent for the lithium metal is also used in this alternative process for the same reasons disclosed in the first-described process. Representative examples of such agents, and amounts used, are also specifically discussed hereinabove, in the first-described process, and need not be reiterated. A preferred solubilizing agent is naphthalene, used in an amount of about 1 to 10 parts by weight per part of lithium metal, and preferably about 1 to 2 parts by weight of lithium metal used.

Temperature in this alternative process can be in the range from about −80°C to +80°C. A preferred process temperature is about 25°C.

The initial molar ratio of metallic lithium to said titanium compound is about 10:1 to 3:1 and preferably about 4:1.

Apparatus for conducting this alternative process can be of any conventional type designed to rigidly exclude elemental oxygen and water while performing the operations of heating, cooling, stirring and filtration. Apparatus which the inventor found useful is the H-shaped apparatus described hereinabove, for the first-described process.

In general, this alternative process comprises the previously described operations, in the first process, of adding the titanium compound and lithium powder into one vertical side of the H-shaped apparatus, under a dry inert atmosphere, and adding diethyl ether to the mixture and cooling the mixture with stirring to a temperature of about −80°C. The mixture is stirred and warmed to about −10°C and a solvating agent for lithium such as naphthalene is then added. The reaction mixture is then stirred and allowed to warm to room temperature and stirring is continued until the red-brown color persists. The resulting mixture is then filtered, in the H-shaped apparatus as previously described; the residue extracted with ether; and the ether filtrate and extracts are combined and then evaporated to yield the redbrown product. The product can be purified by recrystallization from 1,2-dimethoxyethane, diethyl ether or other like solvents, and preferably 1,2-dimethoxyethane.

Yields of product composition in this process are about 15 to 30% of theory based on starting titanium compound.

Advantage can be taken of the fact that the subject compositions are readily soluble in ethereal, aromatic, or alkylamine organic solvents, thus enabling homogeneous catalytic reactions, especially dimerization of ethylene to 1-butene and polymerization of acetylene. It is believed that the reason the subject compositions are highly effective as homogeneous catalysts for such reactions is that a hydrogen atom is attached to the titanium metal in hydride form, and is thus very labile and available for hydride transfer with ethylene to initiate the dimerization reaction, via the formation of a transient anionic titanium (ethylene)ethyl intermediate. In addition, the spatial geometry of the solvated composition in solution seems to promote the hydrogen transfer, and the removal of the 1-butene reaction product.

Accordingly, also a subject of this invention is an improved process for the homogeneous catalytic dimerization of ethylene to 1-butene. The elements of the process with respect to solvent, catalyst concentration, temperature, pressure, reaction time, yield, and apparatus are broadly known.

The improvement over the prior art is the use of the subject compositions, disclosed herein, as homogeneous catalysts for the dimerization. A preferred catalyst for the dimerization is

$$Li(\eta—C_5H_5)(C_5H_5)Ti—H,$$

and a preferred solvent is N,N,N'N'-tetramethylethylenediamine.

The process includes contacting gaseous ethylene with a solution of the subject composition as dimerization catalyst, in an inert solvent therefor, under a pressure of about 1 to 100 atmospheres, at a temperature of about 0° to 100°C. Preferably, a solution of about 1 to 3 parts of the subject composition, as catalyst, in 100 parts by weight of solvent is used, preferably using N,N,N',N'-tetramethylethylene diamine as solvent, at about room temperature, under an atmosphere of gaseous ethylene at a pressure of about 10 atmospheres. The reaction mixture, in a closed pressure vessel, is stirred to contact the liquid and gas phases until the absorption of ethylene by the solution becomes negligible. The product 1-butene is obtaned in about a 90—99% conversion, based on reacted ethylene, and can be isolated and purified by conventional methods such as distillation.

Also in accordance with another aspect of this invention, there is provided a process for producing polyacetylene comprising contacting acetylene gas with a solution consisting essentially of an organic solvent and a dissolved lithium dicyclopentadienyl titanium hydride catalyst of the above described formula, LiRR'TiH.

The acetylene polymerization process of this invention, in general, comprises dissolving the catalyst in a stirred inert organic solvent therefor, optionally containing a lithium cation

chelating agent to aid in dissolving the catalyst in the solvent. Acetylene is then contacted with the solution and absorbed thereby. The polymerization is allowed to proceed until the desired amount of acetylene has been absorbed as indicated by decrease in the reaction pressure. The solvent is then distilled off under reduced pressure and the resulting black residue, being the cryde polyacetylene, can be purified by washing with a suitable solvent. The entire process must be conducted under conditions rigorously excluding the presence of elemental oxygen and water. Conducting the process at temperatures from −110° to −60°C, favors the formation of the cis configuration of polyacetylene and higher temperatures, from 0° to +250°C, favors the formation of the trans configuration. By "doping" with additives as known, these polyacetylenes can be made capable of functioning as electrical conductors or semiconductors.

Representative classes of suitable solvents useful in the process include $C_6$—$C_{14}$ aromatic hydrocarbons, $C_5$—$C_{18}$ linear or branched acyclic or cyclic saturated aliphatic hydrocarbons, $C_4$—$C_6$ cyclic saturated aliphatic mono- or diethers, $C_3$—$C_8$ linear or branched acyclic saturated aliphatic mono- or diethers, $C_7$—$C_{10}$ aromatic mono- or diethers, $C_3$—$C_8$ linear or branched saturated aliphatic tertiary amines, $C_5$—$C_8$ cyclic mono-olefins, $C_2$—$C_{12}$ linear or branched acyclic alpha-olefins, or mixtures thereof.

Representative examples of specific solvents include toluene, hexane, benzene, tetrahydrofuran, dioxane, diethyl ether, 1-2-dimethoxyethane, anisole, n-hexadecane, cyclohexane, triethylamine, cyclohexene, hexene, and the like, or mixtures thereof.

Amount of solvent used in the process is generally not critical.

If the lithium dicyclopendadienyl titanium hydride catalyst is only sparingly soluble in the solvent, as in the saturated aliphatic hydrocarbons, described above, the lithium cation may optionally be chelated to increase the solubility, by use of a chelating agent. Suitable chelating agents include tertiary amines such as N,N,N',N'-tetramethylethylenediamine, cyclic ethers such as 15-crown-5 and 18-crown-6, and cryptates, being bicyclic bridgehead nitrogen diamines, having oxymethylene bridges. Preferred chelating agent is N,N,N',N'-tetramethylethylenediamine.

The amount of said chelating agent used is that amount sufficient to completely dissolve the catalyst in the solvent used and may be as much as two moles chelating agent per mole of catalyst employed.

The amount of catalyst used in the process is not critical. In general, about 1 part catalyst per 1000 to 10,000 parts by weight of solvent, are employed in the process, although higher and lower amounts are also effective.

The amount of acetylene used is dependent upon the size of the apparatus and other factors. The total calculated amount of acetylene, to be used in the reaction, can be present at the beginning of the reaction or acetylene can be continuously introduced into the reaction solution during the process. Various grades of acetylene can be used, commercial, technical, etc., with the proviso that air and moisture and contaminant which might react with the catalyst, such as acetone, should be rigorously excluded from the acetylene feed stream prior to introduction into the reaction solution.

The following examples are illustrative of the best mode of carrying out the invention as contemplated by us and should not be construed as being limitations of the scope and spirit of the instant invention.

Example 1

Synthesis of $Li(\eta—C_5H_5)(C_5H_5)TiH$ from "titanocene" and lithium metal

Into an apparatus consisting of two 500 ml roundbottom flasks connected by a filter frit, was charged (into one flask) 0.4 grams of dry lithium powder (prepared from a lithium dispersion in oil), and 2.9 grams of $\mu$-($\eta^1$:$\eta^5$-cyclopentadienyl)-tris ($\eta$-cyclopentadienyl) dititanium (Ti—Ti) prepared by the procedure described in *J. Am. Chem. Soc. 98,* 8072 (1976), and 0.2 g naphthalene, in the absence of air and moisture. To this was added 300 ml diethyl ether and the mixture was mechanically stirred overnight at room temperature. The mixture was filtered through the frit and the residue further extracted with diethyl until a redbown extract no longer remained. The ether extracts were combined with the filtrate and the ether solvents removed under reduced pressure. A redbrown residue was obtained (yield 0.9 grams). The product was insoluble in aromatic solvents, moderately soluble in ether and 1,2-dimethoxyethane and somewhat more soluble in tetrahydrofuran. An infrared spectrum of the product showed a broad intense band at 1390 $cm^{-1}$, characteristic of the titanium metal-hydride bond.

Example 2

Synthesis of $Li(\eta—C_5H_5)(C_5H_5)TiH$ from titanocene dichloride and lithium metal

Into an apparatus consisting of two 1000 ml. round bottomed flasks, connected by a filter frit, was charged (into one flask) 30 gms. of dicyclopentadienyl titanium dichloride and 3.0 gms. of lithium powder in the absence of air and moisture. A side arm was connected to the same flask and charged with 2.5 gms. of naphthalene. Diethyl ether was distilled into the lithium/dicyclopentadienyl titanium dichloride mixture, and cooled to −80°C. After the ether addition was completed, the mixture was stirred and when temperature reached about −10°C, the naphthalene was added. The flask was cooled with ice water and then allowed to stir and warm spontaneously to room temperature.

Stirring at about 23°C was continued until the solution turned to a green, and then a red-brown color. The solution was filtered and the residue extracted with ether. The ether filtrate and extracts were combined and evaporated in vacuo and the resulting redbrown residue was washed with toluene. A redbrown powder was obtained in a yield of about 4.1 gms. Infrared and nuclear magnetic resonance spectra of the material provided to be identical to that described for the product in Example 1.

Purification and characterization of $Li(\eta—C_5H_5)(C_5H_5)Ti—H$

(Preparation and handling of the above compound was carried out with rigid exclusion of air and moisture). The resulting crude red-brown product from Example 2 (400 mgs.) was loaded into an evacuable H-shaped glass apparatus having vertical sides separated by a horizontal glass tube of the same internal diameter containing a fritted tube. The apparatus allows filtration and recrystallization of air and moisture sensitive materials under reduced pressure. The product was dissolved in 1,2-dimethoxyethane (DME), 50 ml., in the bottom portion of one. vertical side and the resulting solution was then concentrated to a volume of about 40 ml. The redbrown solution was filtered by tilting the apparatus such that the solution flowed from one vertical side through the glass filter to the other vertical side. The solvent was allowed to evaporate off slowly, at about 20°C, by cooling the other vertical side of the H tube to 15°C. A crystalline mass, covered by an oily layer, was seen after about 1 to 2 days of evaporation. The oily portion was washed out with DME and the dry crystalline mass was redissolved. Crystallization resulted after evaporation over several days and afforded 160 mgs of black, brick-shaped crystals of

$Li(\eta—C_5H_5)(C_5H_5)Ti—H$(1,2-dimethoxy-ethane).

Elemental analysis disclosed: Found: C, 61.05, H, 7.42; Ti, 16.10; Li, 2.31, Expected: C, 60.89; H, 7.66; Ti, 17.34; Li, 2.51. A $^{13}C$ nuclear magnetic resonance spectrum in deuterated tetrahydrofuran exhibited a single line at 94.91 ppm downfield (TMS=0 ppm) corresponding to 5 equivalent $C_5H_5$ carbon atoms plus a sequence of lines at 100.82, 102.0, 102.8, 109.1, 110.5 ppm, respectively, for 5 non-equivalent carbon atoms in the ($C_5H_5$) ring. This is basically the same $^{13}C$ spectrum of the $C_5H_5$ rings as shown in Fig. 1 for the tetramethylethylenediamine adduct.

Example 3

A glass reactor was charged with 200 mg. of

$Li(\eta—C_5H_5)(C_5H_5)$ Ti—H,

as prepared by the method of Example 2, and 15 ml N,N,N′,N′-tetramethylethylene diamine. The reactor was charged with ethylene at 10 atm. pressure, and at room temperature. Upon continuing stirring, over a period of about 10 hours, the ethylene pressure decreased to a value of about 3 atmospheres. The product was analyzed by gas chromatography, and shown to be mainly 1-butene.

Example 4

Thirty mg. of crude lithium titanocene hydride (i.e., the subject organotitanium composition above described) obtained from reaction of lithium with dicyclopentadienyl titanium dichloride in ether in accordance with Example 2 above, were dissolved in 250 ml of pure tetrahydrofuran, in the absence of elemental oxygen and water. The solution was stirred and cooled to −80°C. under an atmosphere of 2 liters of acetylene at a pressure of about one atmosphere. Polymerization began almost Immediately upon absorption of acetylene by the stirred solution. The solution was continuously stirred and maintained at −80°C. for 1 hour, then warmed to −35°C. over a 3 to 4 hour period, and then allowed to warm slowly overnight under continuous stirring. The solvent was removed and the resulting polyacetylene was recovered as black shiny lumps in a yield of about 1 to 1.5 grams. Infrared spectral analysis showed the material to be mainly cis-polyacetylene, as evidenced by characteristic absorption band observed at about 740 $cm^{-1}$.

Example 5

The procedure of Example 1 was repeated excepted that the catalyst (30 mg.) was first dissolved in N,N,N′,N′-tetramethylethylenediamine to form a chelate and then excess amine was removed by vacuum distillation. Toluene (500 ml.) was added and the solution was stirred under an atmosphere of acetylene (2 liters) at one atmosphere pressure. The polymerization was carried out at a temperature of about 23—25°C, under agitation, until the uptake of acetylene gas was negligible as evidenced by a constant reaction pressure. Three grams of product were obtained which consisted mainly of the trans form of polyacetylene, as evidenced by the characteristic absorption band observed at about 1015 $cm^{-1}$.

**Claims**

1. An organotitanium composition characterized by the formula: LiRR′TiH, wherein R and R′ are cyclopentadienyl rings of the empirical formula, $C_5H_5$, or are such rings wherein one or more the ring hydrogens is substituted with a substituent inert toward lithium metal, said rings being bonded to titanium by sigma-bonds, pi-bonds and/or mixtures thereof; and said composition exhibiting a $^{13}C$ nuclear magnetic resonance spectrum, in deuterated benzene, in which observed values for the chemical shifts of

the ring carbons in R are different from the observed values for ring carbons in R′; and said composition exhibiting an infrared spectrum in deuterated n-hexadecane in which a titanium metal-hydride absorption band is observed in the region of 1250—1450 $cm^{-1}$.

2. The composition of claim 1 wherein R and R′ are both unsubstituted cyclopentadienyl rings.

3. The composition of claim 1 being an adduct formed with a chelating agent for lithium cation.

4. A process for producing the composition of claim 1 characterized by contacting metallic lithium with a dititanium complex of the formula:

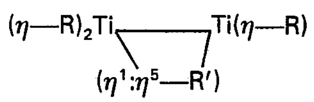

wherein (η—R) radicals are cyclopentadienyl rings, bonded to titanium through five carbon atoms each having the empirical formula, $C_5H_5$, or are such rings wherein one or more of the ring hydrogens is substituted with a substituent inert toward lithium metal, and wherein the ($\eta^1$:$\eta^5$—R′) radical is a cyclopentadienyl ring, bonded to titanium by a mixture of $\eta^1$ and $\eta^5$ bonds, and having the empirical formula, $C_5H_4$, or is such ring wherein one or more of the ring hydrogens is substituted with a substituent inert toward lithium metal; in an inert solvent therefor and in the presence of a polynuclear aromatic hydrocarbon solubilizing agent for lithium, soluble in said solvent; and at a temperature of −10° to +60°C, in the absence of elemental oxygen and water; and with initial molar ratio of metallic lithium to said dititanium complex of 10:1 to 1:1.

5. The process of claim 4 wherein the dititanium complex is μ—($\eta^1$:$\eta^5$-cyclopentadienyl)-tris (η-cyclopentadienyl)dititanium(Ti—Ti), the solvent is diethyl ether, the polynuclear aromatic hydrocarbon is naphthalene, and the product is

$$Li(\eta—C_5H_5)(C_5H_5)TiH.$$

6. A process for producing the composition of claim 1 characterized by contacting metallic lithium with a titanium compound of the formula: $R_2TiX_a$, or dimers thereof, wherein the radicals R are cyclopentadienyl rings, each having the empirical formula, $C_5H_5$, or are such rings wherein one or more of the ring hydrogens is substituted with a substituent inert toward lithium metal; X being halogen, and the subscript *a* being one or two; in diethyl ether solvent and in the presence of a polynuclear aromatic hydrocarbon solubilizing agent for lithium, soluble in diethyl ether; and at a temperature of −80° to +80°C, in the absence of elemental oxygen and water; and with initial molar ratio of metallic lithium to said titanium compound of 10:1 to 3:1.

7. The process of claim 6 wherein said titanium compound is $(C_5H_5)_2TiCl_2$, the solubilizing agent is naphthalene, the temperature is about 25°C and the initial molar ratio of lithium metal to titanium compound is about 4:1.

8. A process for producing 1-butene, comprising contacting ethylene gas with a solution of dimerization catalyst, in an inert solvent therefor, under a pressure of 1 to 100 atmospheres, at a temperature of 0° to 100°C; characterized by using the composition of claim 1 as said catalyst.

9. A process for polymerizing acetylene, comprising contacting acetylene gas under essentially anhydrous, oxygen-free conditions, with a solution consisting essentially of an organic solvent and a dissolved organotitanium catalyst composition at a temperature in the range between minus 120°C and plus 200°C, characterized by providing the composition of claim 1 as said catalyst.

10. Process of claim 9 wherein a chelating agent is used to increase the solubility of the catalyst in the solvent.

**Patentansprüche**

1. Titanorganische Verbindung, gekennzeichnet durch die Formel LiRR′TiH, worin R und R′ Cyclopentadienylringe der empirischen Formel $C_5H_5$ oder solche Ringe, worin eines oder mehrere der Ringwasserstoffatome durch einen gegenüber Lithiummetall inerten Substituenten substituiert sind, bedeuten, wobei diese Ringe durch Sigmabindungen, Pibindungen und/oder Gemische derselben an Titan gebunden sind, und die Verbindung ein $^{13}C$-kernmagnetisches Resonanzspektrum in deuteriertem Benzol hat, in welchem für die chemischen Shifts der Ringkohlenstoffatome in R beobachtete Werte von den für die Ringkohlenstoffatome in R′ beobachteten Werten verschieden sind, und die Verbindung ein Infrarotspektrum in deuteriertem n-Hexadecan hat, in welchem eine Titanmetallhydrid-Absorptionsbande im Bereich von 1250 bis 1450 $cm^{-1}$ beobachtet wird.

2. Verbindung nach Anspruch 1, worin R und R′ beide unsubstituierte Cyclopentadienylringe sind.

3. Verbindung nach Anspruch 1, die ein Addukt ist, welches mit einem Chelatisierungsmittel für das Lithiumkation gebildet ist.

4. Verfahren zur Herstellung der Verbindung nach Anspruch 1, gekennzeichnet durch Behandlung von metallischem Lithium mit einem Dititankomplex der Formel

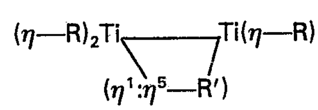

worin die (η—R)-Reste Cyclopentadienylringe, die über 5 Kohlenstoffatome an Titan gebunden sind und die jeweils die empirische Formel $C_5H_5$ haben, oder solche Ringe, worin eines oder

mehrere der Ringwasserstoffatome durch einen gegenüber Lithiummetall inerten Substituenten substituiert sind, bedeuten und worin der ($\eta^1$:$\eta^5$—R')-Rest ein Cyclopentadienylring, der an Titan über ein Gemisch von $\eta^1$- und $\eta^5$-Bindungen gebunden ist und die empirische Formel $C_5H_4$ hat, oder ein solcher Ring, worin eines oder mehrere der Ringwasserstoffatome durch einen gegenüber Lithiummetall inerten Substituenten substituiert sind, ist, in einem inerten Lösungsmittel hierfür und in Gegenwart eines in diesem Lösungsmittel löslichen mehrkernigen aromatischen Kohlenwasserstoffes als löslichmachendes Mittel für Lithium und bei einer Temperatur von —10 bis 60°C in Abwesenheit von elementarem Sauerstoff und Wasser und mit einem Anfangsmolverhältnis von metallischem Lithium zu dem Dititankomplex von 10:1 bis 1:1 behandelt.

5. Verfahren nach Anspruch 4, worin der Dititankomplex $\mu$—($\eta^1$:$\eta^5$ - Cyclopentadienyl) - tris - ($\eta$ - cyclopentadienyl) - dititan - (Ti—Ti) ist, das Lösungsmittel Diäthyläther ist, der mehrkernige aromatische Kohlenwasserstoff Naphthalin ist und das Produkt

$$Li(\eta—C_5H_5)(C_5H_5)TiH$$

ist.

6. Verfahren zur Herstellung der Verbindung nach Anspruch 1, dadurch gekennzeichnet, daß man metallisches Lithium mit einer Titanverbindung der Formel $R_2TiX_a$ oder Dimeren derselben, worin die Reste R Cyclopentadienylringe jeweils mit der empirischen Formel $C_5H_5$ oder solche Ringe, worin eines oder mehrere der Ringwasserstoffatome durch einen gegenüber Lithiummetall inerten Substituenten substituiert sind, X Halogen ist und a 1 oder 2 bedeutet, in Diäthyläther als Lösungsmittel und in Gegenwart eines in Diäthyläther löslichen mehrkernigen aromatischen Kohlenwasserstoffes als löslichmachendes Mittel für Lithium bei einer Temperatur von —80 bis 80°C in Abwesenheit von elementarem Sauerstoff und Wasser und mit einem Anfangsmolverhältnis von metallischem Lithium zu der Titanverbindung von 10:1 bis 3:1 behandelt.

7. Verfahren nach Anspruch 6, worin die Titanverbindung $(C_5H_5)_2TiCl_2$ ist, das löslichmachende Mittel Naphthalin ist, die Temperatur bei etwa 25°C liegt und das Anfangsmolverhältnis von Lithiummetall zu Titanverbindung etwa 4:1 ist.

8. Verfahren zur Herstellung von 1-Buten durch Behandlung von Äthylengas mit einer Dimerisierungskatalysatorlösung in einem inerten Lösungsmittel hierfür unter einem Druck von 1 bis 100 at bei einer Temperatur von 0 bis 100°C, gekennzeichnet durch die Verwendung der Verbindung nach Anspruch 1 als diesen Katalysator.

9. Verfahren zur Polymerisierung von Acetylen durch Behandlung von Acetylengas unter im wesentlichen wasserfreien, sauerstofffreien Bedingungen mit einer Lösung, die im wesentlichen aus einem organischen Lösungsmittel und einem gelösten titanorganischen Katalysator besteht, bei einer Temperatur im Bereich zwischen —120°C und 200°C, dadurch gekennzeichnet, daß man als den Katalysator die Verbindung nach Anspruch 1 verwendet.

10. Verfahren nach Anspruch 9, worin ein Chelatisierungsmittel verwendet wird, um die Löslichkeit des Katalysators in dem Lösungsmittel zu erhöhen.

**Revendications**

1. Composition d'organotitane, caractérisée par la formule: LiRR'TiH où R et R' sont des noyaux de cyclopentadiényle de formule empirique $C_5H_5$, ou sont des noyaux où un ou plusieurs des hydrogènes du noyau sont substitués par un substituant inerte vis-à-vis du lithium métallique, les noyaux étant liés au titane par des liaisons sigma, des liaisons pi et/ou leurs mélanges; et la composition présentant un spectre de résonance magnétique nucléaire de $C^{13}$ dans du benzène deutéré, dans lequel les valeurs observées pour les déplacements chimiques des carbones du noyau dans R sont différentes des valeures observées pour les carbones du noyau dans R'; et la composition présentant un spectre infrarouge dans du n-hexane deutéré dans lequel une bande d'absorption en titane métallique-hydrure est observée dans la région de 1250—1450 $cm^{-1}$.

2. Composition selon la revendication 1, caractérisée en ce que R et R' sont tous deux des noyaux de cyclopentadiényle non substitués.

3. Composition selon la revendication 1, caractérisée en ce qu'elle est un produit d'addition formé avec un agent de chélation pour le cation lithium.

4. Procédé pour produire la composition de la revendication 1, caractérisé en ce qu'on met en contact du lithium métallique avec un complexe de dititane ayant la formule:

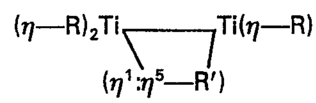

où les radicaux ($\eta$—R) sont des noyaux de cyclopentadiényle, liés au titane par l'intermédiaire de cinq atomes de carbone, chacun ayant la formule empirique $C_5H_5$ ou sont ces noyaux où un ou plusieurs des hydrogènes du noyau sont substitués par un substituant inerte vis-à-vis du lithium métallique, et où le radical ($\eta^1$:$\eta^5$—R') est un noyau de cyclopentadiényle, lié au titane par un mélange de liaison $\eta^1$ et $\eta^5$ et ayant la formule empirique $C_5H_4$, ou est ce noyau où un ou plusieurs des hydrogènes du noyau sont substitués par un substituant inerte vis-à-vis du lithium métallique; dans un solvant inerte en en présence d'un agent de solubilisation formé d'hydrocarbure aromatique polynucléaire pour le lithium, soluble dans ce solvant; et à une température de −10 à +60°C en l'absence d'oxygène élémentaire et d'eau et avec un rapport molaire initial entre le lithium métallique et le complexe de dititane de 10:1 à 1:1.

5. Procédé selon la revendication 4, caractérisé en ce que le complexe de dititane est $\mu$—($\eta^1$:$\eta^5$ - cyclopentadiényl) - tris($\eta$ - cyclopentadiényl)dititane(Ti—Ti), le solvant est de l'éther diéthylique, l'hydrocarbure aromatique polynucléaire est le naphtalène et le produit est

$$Li(\eta{-}C_5H_5)(C_5H_5)TiH.$$

6. Procédé de production de la composition de la revendication 1, caractérisé en ce qu'on met en contact du lithium métallique avec un composé de titane ayant la formule: $R_2TiX_a$, ou ses dimères, où les radicaux R sont des noyaux de cyclopentadiényle, chacun ayant la formule empirique, $C_5H_5$ ou représente ces noyaux où un ou plusieurs des hydrogènes du noyau sont substitués par un substituant inerte vis-à-vis du lithium métallique; X étant un halogène et l'indice *a* valant un ou deux; dans de l'éther diéthylique comme solvant et en présence d'un agent de solubilisation formé d'hydrocarbure aromatique polynucléaire pour le lithium, soluble dans l'éther diéthylique; et à une température de −80 à +80°C, en l'absence d'oxygène élémentaire et d'eau; et avec un rapport molaire initial entre le lithium métallique et le composé de titane de 10:1 à 3:1.

7. Procédé selon la revendication 6, caractérisé en ce que le composé de titane est $(C_5H_5)_2TiCl_2$, l'agent de solubilisation est le naphtalène, la température est environ 25°C et le rapport molaire initial entre le lithium métallique et le composé de titane est environ 4:1.

8. Procédé de production de 1-butène, caractérisé en ce qu'il consiste à mettre en contact de l'éthylène gazeux avec une solution de catalyseur de dimérisation, dans un solvent inerte, sous une pression de 1 à 100 atmosphères, à une température de 0 à 100°C caractérisé en ce qu'on utilise la composition de la revendication 1 comme catalyseur.

9. Procédé de polymérisation d'acétylène consistant à mettre en contact de l'acétylène gazeux dans des conditions essentiellement anhydres, exemptes d'oxygène, avec une solution se composant essentiellement d'un solvent organique et d'une composition catalytique d'organotitane dissoute à une température dans l'intervalle compris entre moins 120°C et plus 200°C, caractérisé en ce qu'on prévoit la composition de la revendication 1 en tant que catalyseur.

10. Procédé selon la revendication 9, caractérisé en ce qu'un agent de chélation est utilisé pour augmenter la solubilité du catalyseur dans le solvant.

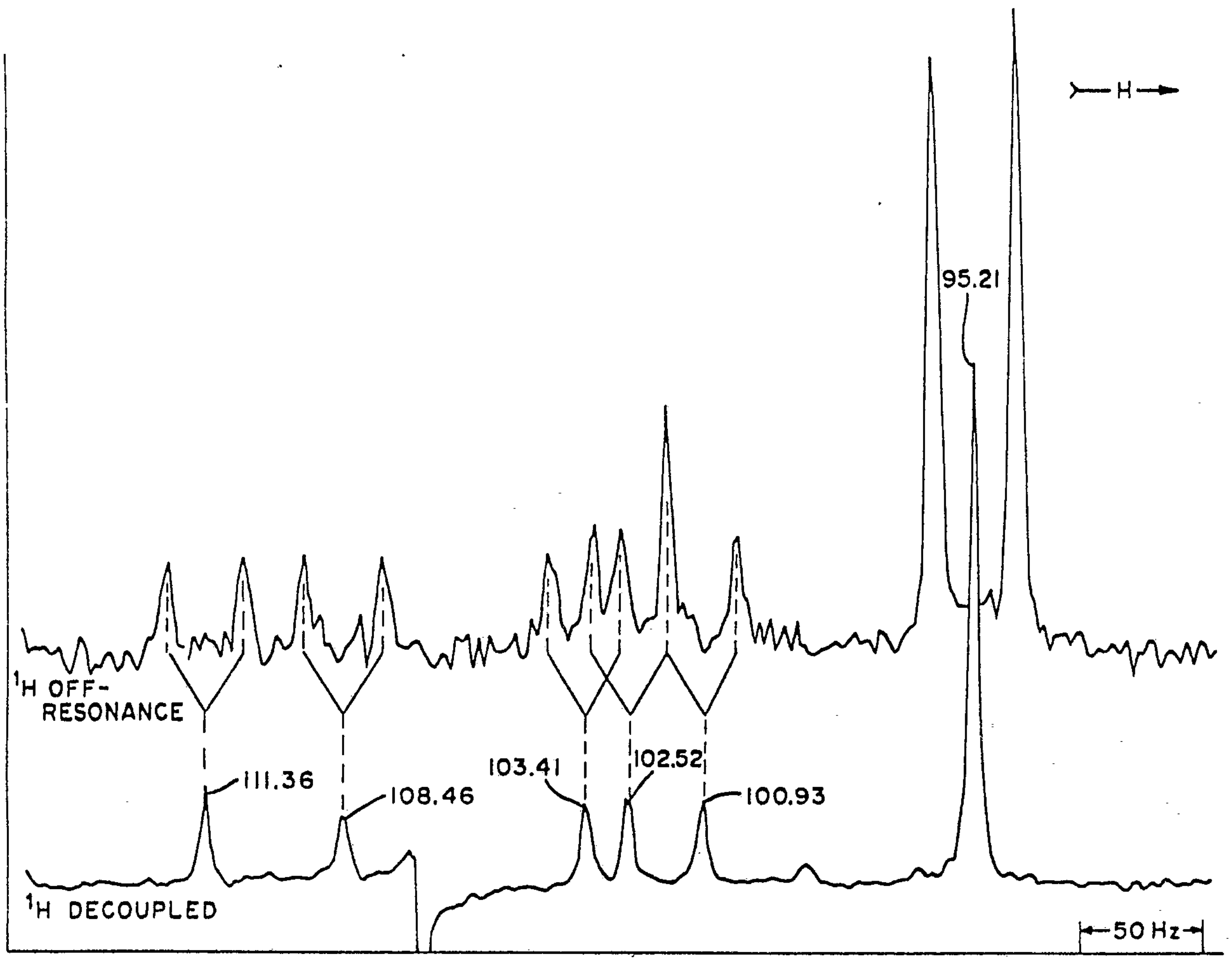

>—H→
95.21
$^1$H OFF-RESONANCE
111.36
108.46
103.41
102.52
100.93
$^1$H DECOUPLED
←50 Hz→


FIG. 1

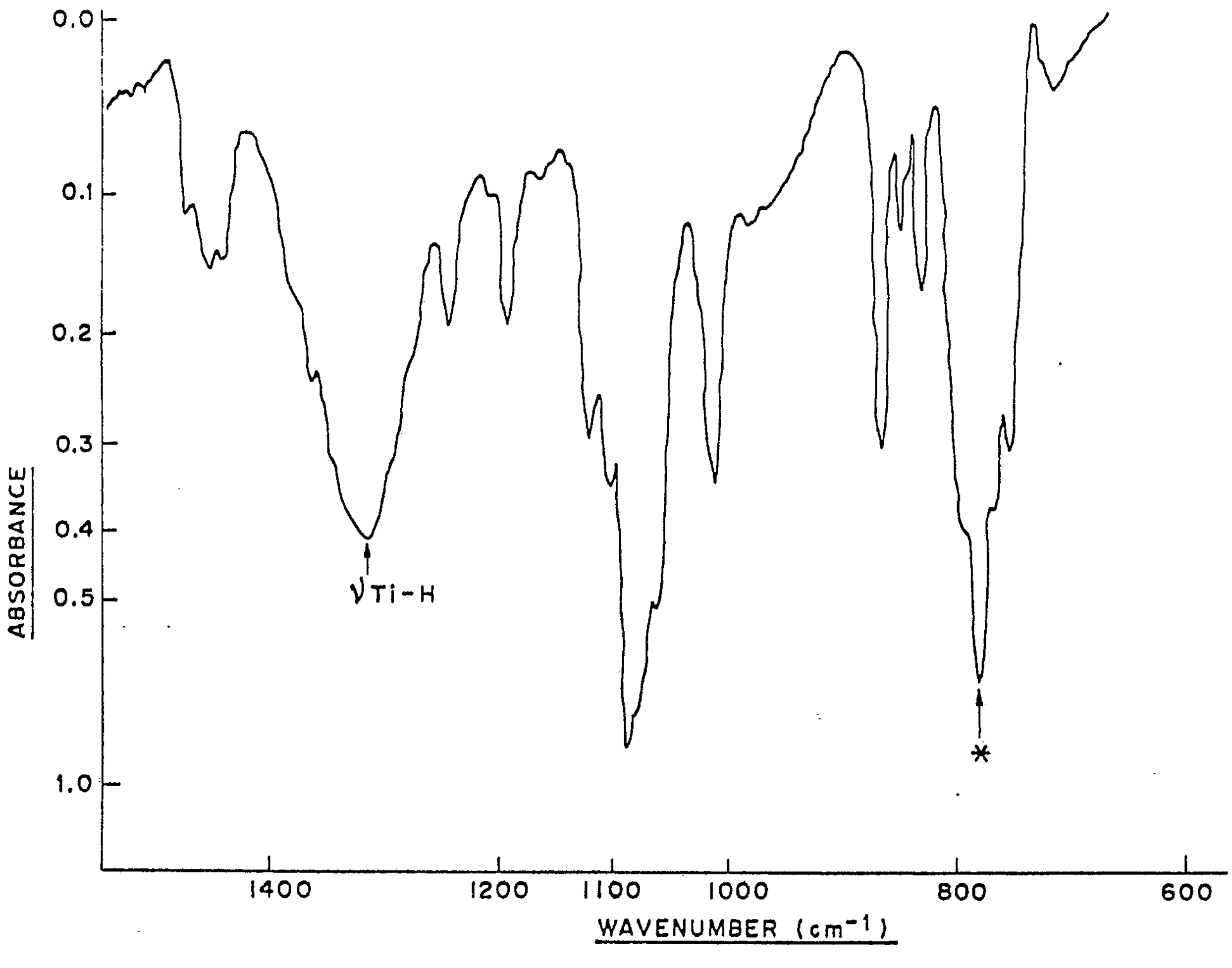
0.0
0.1
0.2
0.3
0.4
0.5
1.0
ABSORBANCE
νTi-H
*
1400
1200
1100
1000
800
600
WAVENUMBER (cm-1)

FIG. 2